**Europäisches Patentamt**

**European Patent Office**

**Office européen des brevets**

(11) Publication number: **0 169 339**

**A1**

(12) **EUROPEAN PATENT APPLICATION**

(21) Application number: **85106596.1**

(22) Date of filing: **29.05.85**

(51) Int. Cl.⁴: **G 01 C 21/22**
**G 09 B 29/10**

(30) Priority: **27.06.84 JP 131072/84**

(43) Date of publication of application:
**29.01.86 Bulletin 86/5**

(84) Designated Contracting States:
**DE FR GB SE**

(71) Applicant: **HITACHI, LTD.**
**6, Kanda Surugadai 4-chome Chiyoda-ku**
**Tokyo 100(JP)**

(72) Inventor: **Shirai, Hiroshi**
**Kozanryo A-207, 1034**
**Higashiishikawa Katsuta-shi(JP)**

(72) Inventor: **Endo, Akiro**
**5-48, Bizenmachi**
**Mito-shi(JP)**

(72) Inventor: **Shibata, Takanori**
**15-2, Onumacho-4-chome**
**Hitachi-shi(JP)**

(72) Inventor: **Tsujii, Fumio**
**1060-2, Suifucho**
**Mito-shi(JP)**

(72) Inventor: **Matsuoka, Yoji**
**13, Murata-Apartment House**
**1199-5, Senbacho Mito-shi(JP)**

(74) Representative: **Patentanwälte Beetz sen. - Beetz jun.**
**Timpe - Siegfried - Schmitt-Fumian**
**Steinsdorfstrasse 10**
**D-8000 München 22(DE)**

(54) **Navigator system for motor vehicle.**

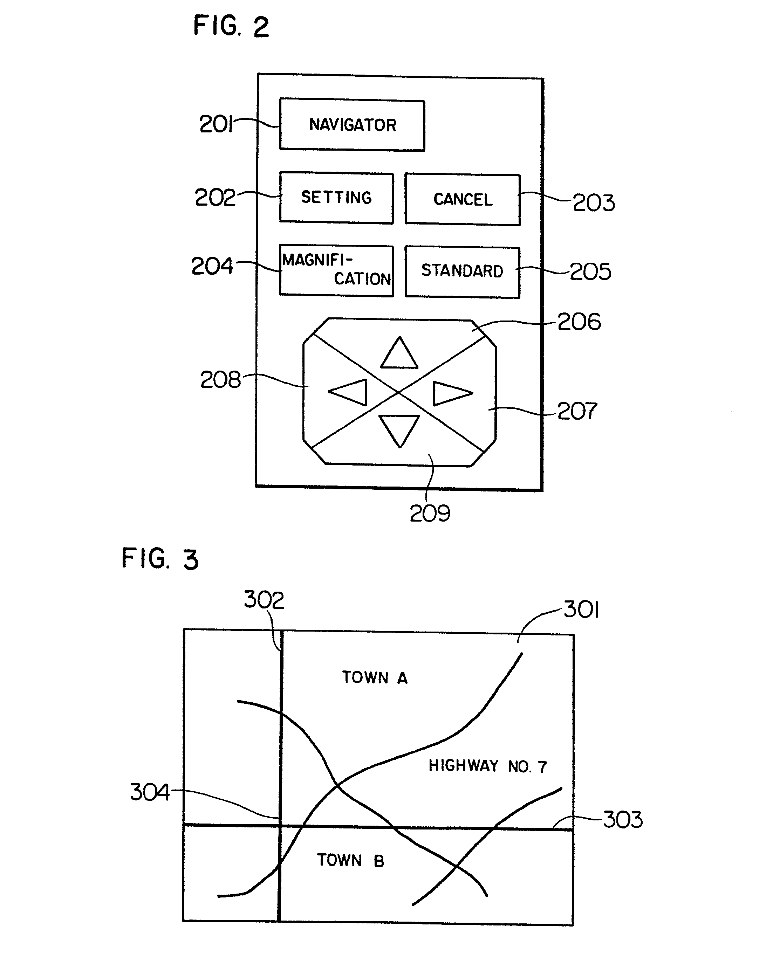

(57) A navigator system for a motor vehicle comprises a vehicle speed sensor (102) provided for determining a travel distance of the vehicle on the basis of the speed thereof, an azimuth sensor (101) for detecting the azimuth of the travelling direction of the vehicle, a cassette tape device (105) for storing digital road map information of a plurality of predetermined regions, a controller (100) for selecting a map of the desired region from the map information stored in the cassette tape device, determining the initial location of the vehicle in the selected map and updating the locating of the vehicle in the displayed map on the basis of the data fetched from the outputs of the vehicle speed sensor and the azimuth sensor, as the vehicle travels, a manipulating switch panel (Figure 2) for commanding the selection of the map and determination of the initial location of the vehicle, and a display unit (116) for displaying visually the selected map, the initial location of the vehicle and the displacement of the vehicle through graphic memory means. The road map data stored in the cassette tape device contain in addition to the data of standard maps at least the data which indicate the presence or absence of adjacent map and the number of magnified maps together with data of the magnified map. The manipulating switch panel include switches (202, 204) for selecting one of the standard map and the magnified map and is adapted to display whether a magnified map is available for the region in which the vehicle is currently located.

./...

## FIG. 1

## NAVIGATOR SYSTEM FOR MOTOR VEHICLE

BACKGROUND OF THE INVENTION

The present invention relates generally to a navigator system for a motor vehicle and more particularly to a navigator system for a motor vehicle adapted to display a road map and a travel path along which the vehicle has traveled on a display mounted on the vehicle.

As the navigator system for the motor vehicle, there has been heretofore known a system capable of exchanging automatically adjacent maps with one another (reference may be made, for example, to Japanese Patnet Application Laid-Open No. 206817/1983). In the hitherto known system of this type, when a plurality of adjacent maps are available, one of them is selectively determined in dependence on the traveling direction of the motor vehicle for updating automatically the display of the map. However, the known navigator system is incapable of informing operator of the vehicle the presence or absence of the desired adjacent map and the number of the available adjacent maps. Further, no measures are provided for the region for which no map has been prepared available.

On the other hand, for maneuvering the motor vehicle in a great city where the road network is dense and complicated, the map of a standard reduced scale (hereinafter also referred to as the standard map) can not assure adequate visual recognizability. Accordingly, for realizing the inherent function of the navigator system, it is desirable that the standard map can be exchanged with a magnified map.

In this connection, it is conceivable to equip the system with an external storage device for storing data of the magnified maps. However, when a cassette tape recorder or the like which requires the sequential access is used to this end, a lot of time will be involved in making access to the storage location or address at which a desired road map data are stored to fetch data. As a result, exchange of maps to be displayed can not be realized at a high speed, degrading the performance of the navigator system, to disadvantages.

SUMMARY OF THE INVENTION

It is an object of the present invention to provide a navigator system for a motor vehicle in which the shortcomings of the prior art system described above are eliminated and which can carry out selection and display of a desired map with high reliability and allows exchange of displayed road maps of different reduced scales (i.e. different diminuation ratios) to be effected at a high speed.

In view of the above object, it is proposed according to an aspect of the invention that when a motor vehicle is outside of a road map being currently displayed, check is made as to whether a map which lies adjacent to the map being displayed as viewed in the traveling direction of the vehicle is prepared available or not. If not, the absence of the adjacent map is given as visual information. In case a number of adjacent maps are availble, one of them is selected to be displayed in exchange with the map being displayed. Further, it is checked whether maps of reduced scales differing from that of the map being currently displayed and encompassing the current location of the vehicle are available or not. In the former case, the presence or availability of the maps of different reduced scales are informed to the operator or driver whereby the exchange of displays of the road maps can be effected repidly in accordance with the request of the driver. More specifically, data of the presence of the adjacent maps, the number thereof as well as the presence of magnified maps and coordinates representative of positions thereof are added to the road map data. In that case, the adjacent map is prepared greater than the region covered by the adjacent map when it is displayed, so that the adjacent maps peripherally superpose one another. Dur to this feature, it is possible to index previously the road map data of the adjacent map to be next displayed when the vehicle is located in the superposed or overlap area, whereby the change-over of the display to the adjacent map can be effected repidly. This feature can be applied to the display of the magnified map.

BRIEF DESCRIPTION OF THE DRAWINGS

Fig. 1 is a view showing a general arrangement of the navigator system according to an embodiment of the present invention;

Fig. 2 is a view showing a manipulating switch array employed in the system shown in Fig. 1;

Fig. 3 is a view showing an example of the map information displayed on a color CRT of the system shown in Fig. 1;

Figs. 4 and 5 are views showing map information produced on the color CRT display for illustrating operation of the system according to the invention;

Fig. 6 is a view for illustrating a map recording schema adopted in the system shown in Fig. 1;

Fig. 7 is a view for showing data format according to an embodiment of the invention; and

Figs. 8 and 9 are flow charts for illustrating operation of the navigator system according to an embodiment of the invention.

DESCRIPTION OF THE PREFERRED EMBODIMENTS

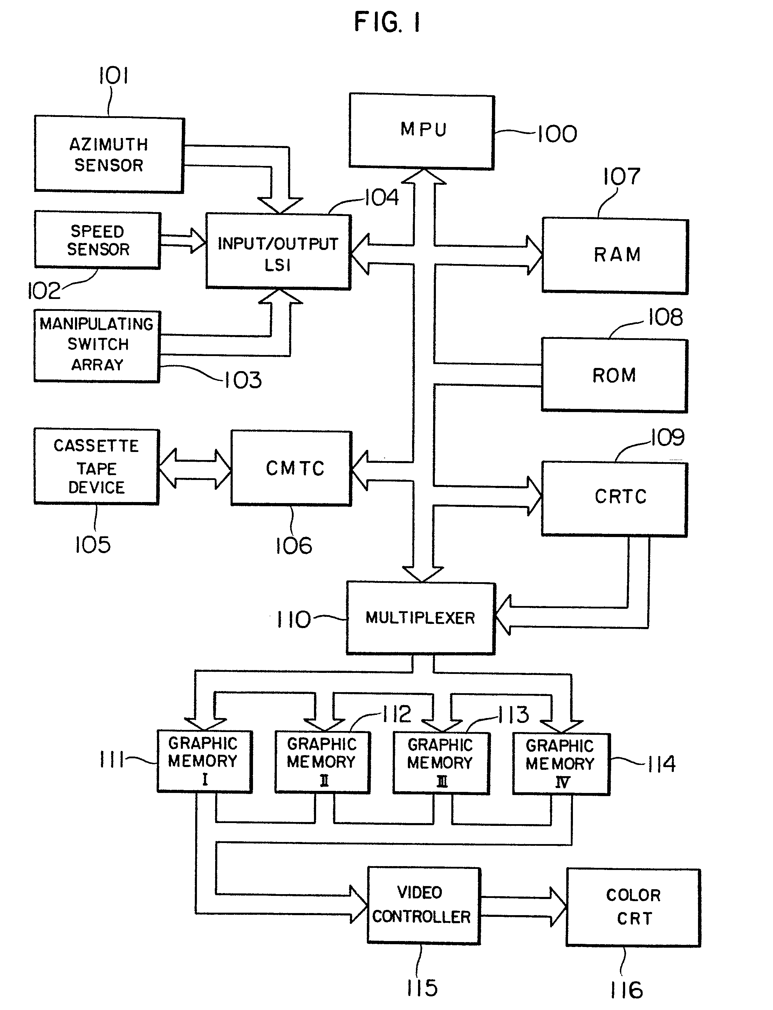

In the following, the present invention will be described in detail in conjunction with the exemplary embodiments thereof by referring to the accompaying drawings. Referring to Fig. 1 which shows a general arrangement of the navigator system according to an embodiment of the invention, operation of the whole navigator system is performed under the control of a

0169339

micro-computer (hereinafter also referred to as MPU in abbreviation) 100. A reference numeral 101 denotes an azimuth sensor device for detecting the absolute azimuth or bearing of a vehicle in the traveling direction thereof by making use of terrestrial magnetism to produce as the output signal thereof analogue quantities representative of X- and Y-components of the detected aximuth or bearing after amplification. A vehicle speed sensor 102 produces a pulse signal having a repetition rate representative of the vehicle speed.

A reference numeral 103 denotes a manipulating switch array or panel for allowing transactions or communication between the MPU 100 and operator or driver of the vehicle. This switch array will be described in detail hereinafter in conjunction with Fig. 2. A numeral 104 denotes an input/output LSI (large-scale integrated circuit) serving as interface among the MPU 100, the azimuth or bearing sensor 101, the vehicle speed sensor 102 and the manipulating switch array 103.

A reference numeral 105 denotes an external storage or memory equipment for storing digital data of road maps prepared for display on a display screen. The memory equipment may be constituted, for example, by a cassette tape recorder.

A numeral 106 denotes a magnetic tape recorder control LSI (also referred to as CMTC in abbreviation) for controlling the input/output or transfer of control signals and data between the MPU 100 and the cassette

0169339

tape recorder 105.

A numeral 107 denotes a volatile memory (random access memory or RAM) serving for storing temporarily data upon execution of arithmetic and control operations by the MPU 100.

A numeral 108 denotes a nonvolatile memory (read-only memory or ROM) for storing programs for various control operations and arithmetic operations to be executed by the MPU 100.

A numeral 109 denotes a CRT controller LSI (referred to as CRTC in abbreviation) which constitutes the heart of a circuitry for transmitting various data such as those of road maps to a color CRT (cathode-ray tube) display 116.

A numeral 110 denotes a multiplexer serving for changing over interconnections between memories 111 to 114 for storing contents or data to be displayed on the color CRT 116 and the MPU 100, the CRTC 109 and others.

Numerals 111 to 113 denote graphic memories for storing data of road maps transferred thereto from the cassette tape recorder 105. More specifically, the road map data are read out from the cassette tape 105 through the CMTC 106 under the control of the MPU 100, wherein R-data (i.e. data to be displayed in red), G-data (i.e. data to be displayed in green) and B-data (i.e. data to be diapleyed in bule) are, respectively, written in the memories 111, 112 and 113 by way of the multiplexer 110.

A numeral 114 denotes a graphic memory serving for storing the travel path of the vehicle which can be determined on the basis of the initial or start location of the vehicle derived from the manipulating switch array 103, the travel direction of the vehicle derived from the output of the azimuth or bearing sensor 101 and the travel distance of the vehicle derived from the output of the vehicle speed sensor 102.

A reference numeral 115 denotes a video controller for performing level conversion and waveform conditioning to assure compatibility of the color CRT 116 of a particular specification. The video controller is connected to the graphic memories 111 to 114 and the color CRT 116 for transferring the data of the memories 111 to 114 to the CRT 116 to display on the screen thereof the road map and the travel path of the vehicle.

Fig. 2 shows an exemplary arrangement of the manipulating switch array or panel. In the figure, a reference numeral 201 denotes a switch for activating the function of navigation. This switch may be constitued by a push-button type lighting switch (e.g. a surface illuminating LED tact switch) which is lit by a first push but put out by a further push.

Upon actuation of the navigation switch 201, the initial value "0" of the map number is loaded in the memory 114 from the ROM 108 and displayed on the color CRT 116 through the video controller 115.

Operator finds out the number of the map which contains the current location of the vehicle through retrieval of a map file prepared previously. Subsequently, the number of numeral displayed on the CRT 116 is varied by actuating key switches 206 to 209 of the switch panel. In this connection, the keys 206 and 208 are destined to increment the displayed numeral while the keys 207 and 209 are destined for decrementing the displayed numeral. Throught appropriate manipulation of these keys 206 to 209, the map number displayed on the CRT 116 is caused to coincide with the identified map number of the map file.

In succession to the establishment of coincidence, a setting switch 202 is pushed, whereupon the MPU 100 reads out data of the corresponding map from the cassette tape recorder 105 through then CMTC 106 and stores the road map data (i.e. the R-, G- and B-data) in the graphic memories 111, 112 and 113, respectively, through the multiplexer 110, which data are then displayed on the color CRT 116 through the video controller 115.

Subsequently, for determining the current location of the vehicle on the map being displayed, cursol data is written in the memory 114 from the ROM 108 in a manner illustrated in Fig. 3.

In Fig. 3, a reference numeral 301 denotes generally the map reproduced from the data read out from the cassette tape 105 through the procedure described above. Numerals 302 and 303 designate cursors intersecting each other to constitute a cross-cursor. The intersection 304 of the cross-cursor is moved to the point corresponding to the current location of the vehicle. The movement of the vertical cursor 302 can be effected by actuating the key switches 208 and 207 shown in Fig. 2. More specifically, actuation of the switch 208 moves the vertical cursor 302 to the left as viewed in Fig. 3, while the cursor 302 is moved to the right by actuating the switch 207. On the other hand, movement of the horizontal cursor 303 is effected with the aid of the key switches 206 and 209. By actuating the key switch 206, the horizontal cursor 303 is moved upwardly while it is moved downwardly in response to actuation of the key 209.

When the intersection 304 has been positioned at the point corresponding to the current location of the vehicle through the manipulation of the switches 208 and 207, the setting key 202 is actuated to inform the MPU 100 that the cursor positioning procedure has been completed. A reference numeral 203 denotes a cancel key switch serving to inform the MPU 100 of the cancelling of the map number and the current vehicle location as set.

In Fig. 2, a numeral 204 designates a push-button type lighting switch which is lit when the vehicle enters a region for which a magnified map is previously prepared available in the state where the navigator system is operating. When the switch 204 is pushed in the lighting state thereof, the map being displayed is changed over to the corresponding magnified map, whereupon the light is put out. A numeral 205 designates a push-button type lighting switch which is lit upon changing-over to the magnified map to indicate that the map of the standard reduced scale (i.e. the map displayed before the exchange with the magnified map) is ready to be displayed. When the switch 205 is pushed in the lighting state thereof, the display on the color CRT is again changed over to the map of the standard reduced scale from the magnified map. Then, the illumination of the switch 205 is put out, while the switch 204 for the magnification is lit.

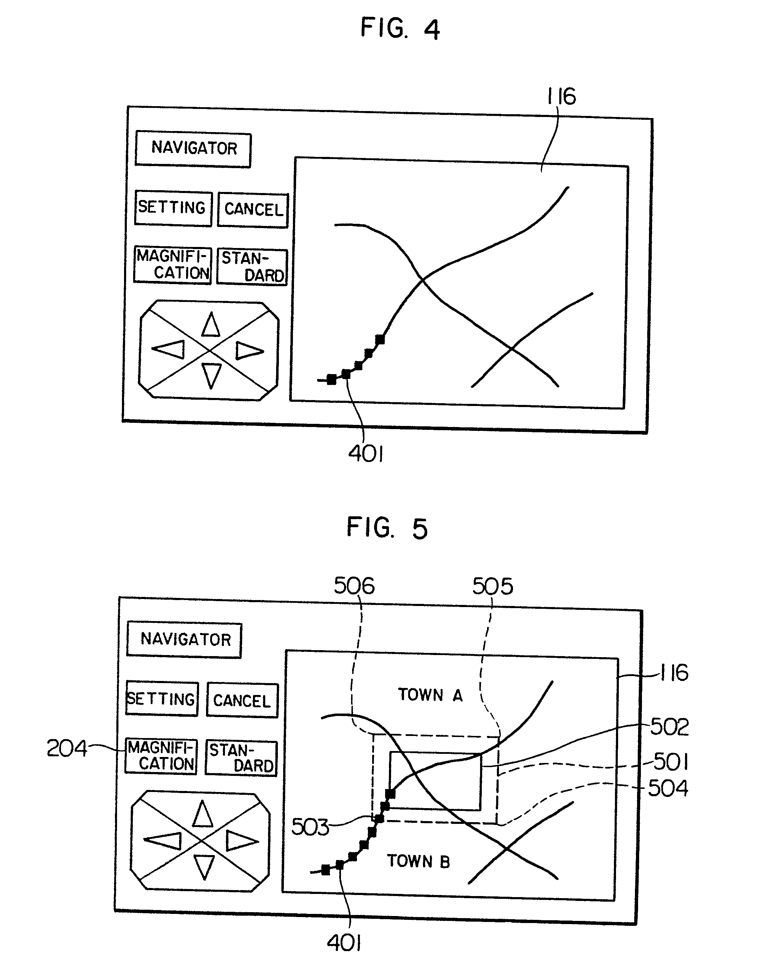

Fig. 4 shows an example of the display produced by the naviagor system according to the invention.

It will be seen in Fig. 4 that a road map (of a standard reduced scale) is displayed on the color CRT 116 with the travel path 401 which the vehicle followed being displayed in superimposition. Referring to Fig. 5, there is illustrated a display on the color CRT 116 which shows a manner in which magnified maps of a region or regions covered by the standard map previously prepared are displayed. More specifically, a rectangular block 502 as displayed on the color CRT 116 indicates that the magnified map for the region enclosed by the block 502 is previously prepared available for display, while another rectangular block 501 depicted by a broken line indicates that a map for a region 501 which covers or encompasses the region 502 is also prepared available for display. The region 501 is referred to

- 11 -

0169339

as the encompassing region or map.

When the vehicle following the travel path 401 enters the region 502 for which the magnified map is available for the display, the switch 204 for the magnification is lit.

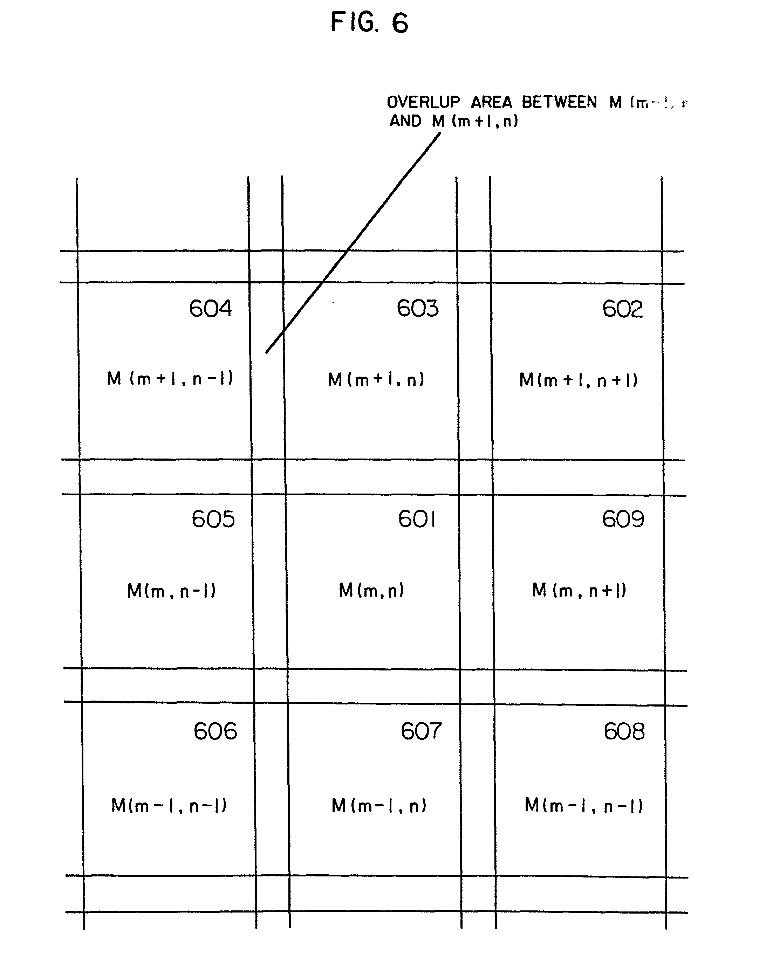

Fig. 6 shows a manner in which the map data are stored in the navigator system according to the present invention. Relation between a particular region and adjacent regions will be seen in this figure.

Referring to Fig. 6, in dependence on the overlap area between the adjacent regions in which the vehicle following the travel path 401 has entered, determination is made as to the map to which the display on the CRT csreen is to be updated while examining the presence or absence of a map for a region located adjacent to the region to be updated. Further, when the vehicle enters the sub-region for which the magnified map is prepared available, the display can be changed over to the magnified map, as described hereinbefore in conjunction with Fig. 5. The changing-over should preferably be accomplished at as high a rate as possible. To this end, corresponding data have to be added to the map data.

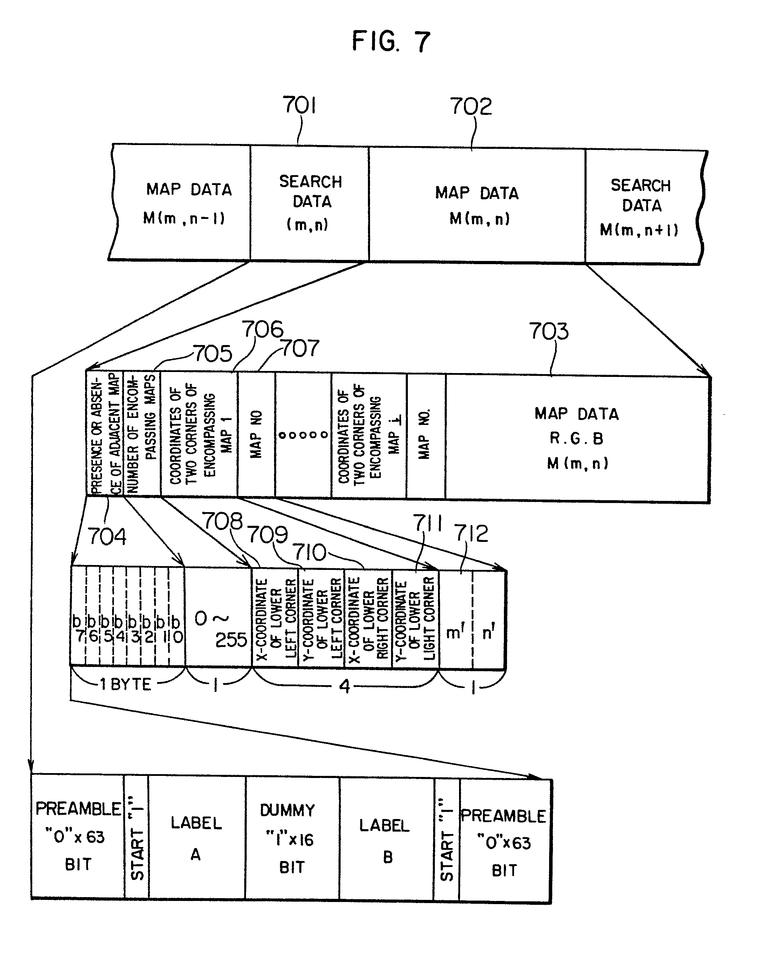

Fig. 7 illustrates a format in which the map data are stored on the cassette tape.

Referring to the figure, a map chart data generally denoted by 702 is composed of search data 701 and the map data 703 in the inherent sense, wherein the map data 703 is added with various data such as those indicated by 704 etc..

The search data 701 is composed of a preamble section including 63 bits of "0", a start section including a single bit of "1", a label-A section recording the search data used for searching actually a map, a dummy section including 16 bits of "1", a label-B section for recording the search data, another start section and another preamble section of 63 bits of "0". Data of the map identifying number is recorded in both the label-A section and the label-B section in the directions opposite to each other. In other words, the search data is formated symmetrically about the dummy section. This is for the purpose of making it possible to read out the map number data irrespective of whether the tape is transported in the forward direction or in the backward direction. As the alternative to the symmetrical formating of the search data, it is also possible to change the sequence of reading the data in dependence on the direction in which the tape is transported. In the latter case, the search data field may be composed of the preamble section, the start section, the label-A section, the start section and the preamble section in this sequence, wherein the search data contained in the label-A section may be read out starting from the least significant bit or LSB when the tape is transported in the forward direction while the search data is read out starting from the most significant bit or MSB when the tape is transported in the backward direction.

In this manner, the searching of a desired map can be accomplished speedily due to the capability of searching the map identifying number contained in the label section at a high speed. With the aid of the map identifying number, the desired map data can be searched out.

Next, details of the map data field 702 will be described. Referring to Fig. 7, a numeral 704 denotes an identifier section indicating whether maps for regions located adjacent to the map being displayed are prepared available or not. This identifier section includes eight bits (one byte) $b_0$, $b_1$, ..., $b_7$ which are allotted to the maps to be located around and adjacent to the map now being displayed sequentially counterclockwise starting from the map located at the upper right corner of the map being displaced. When "1" is set at the bits, this means that the associated adjacent maps are present.

More specifically, assuming that the area 601 shown in Fig. 6 is a standard map being displayed, the bits $b_0$, $b_1$, $b_2$, $b_3$, $b_4$, $b_5$, $b_6$ and $b_7$ are correspondingly allotted to the adjacent maps 602, 603, 604, 605, 606, 607, 608 and 609, respectively. The bit $b_i$ (where i = 0, ..., or 7) of "1" means that the corresponding adjacent map is present. The data stored in the section 705 indicates how many magnified maps are available for the standard map M (m, n). Sections 706 and 707 store the data of the magnified maps. As will be seen from the contents stored in areas 708, 709, 710 and 711, the section 706 stores the data of positions of the magnified maps relative to the associated standard map M (m, n). By way of example, referring to Fig. 5, the area 708 stores the X-coordinate of the lower left corner 503 of the encompassing map 501, the area 709 stores the Y-coordinate of the lower left corner 503, the area 710 stores the X-coordinate of the lower right corner 504, and the area 711 contains the Y-coordinate of the lower right corner 504. Since the coordinates of the corner points 505 and 506 can be arithmetically determined on the basis of the cordinate data of the corners 503 and 504 on the condition that the magnified or encompassing map 501 is of a predetermined form, it is unnecessary to provide the coordinate data for the corners 503 and 504.

The section 707 contains the map number data for identifying the magnified or encompassing map stored in the section 706, as will be seen in areas 712. In accordance with this map identifying number, the data of the corresponding magnified encompassing map are read out from the cassette tape recorder 105 by using the search data. The data format of the magnified map is generally designated by a numeral 703.

When a further magnified map (referred to as the detail map) is be available for a given mangified map, the detailed map data may be arranged in a format similar to that 702 of the magnified or encompassing map except that the "encompassing map" of the data placed in the sections 704 etc. is replaced by "the detailed map". In this connection, a switch labelled "DETAIL" may be additionally provided in the switch array shown in Fig. 2. In this manner, magnification of a map may be effected at a plurality of steps. Further, data for the magnified maps inclusive of the detailed map are prepared for the number of these maps which is stored at the section 705.

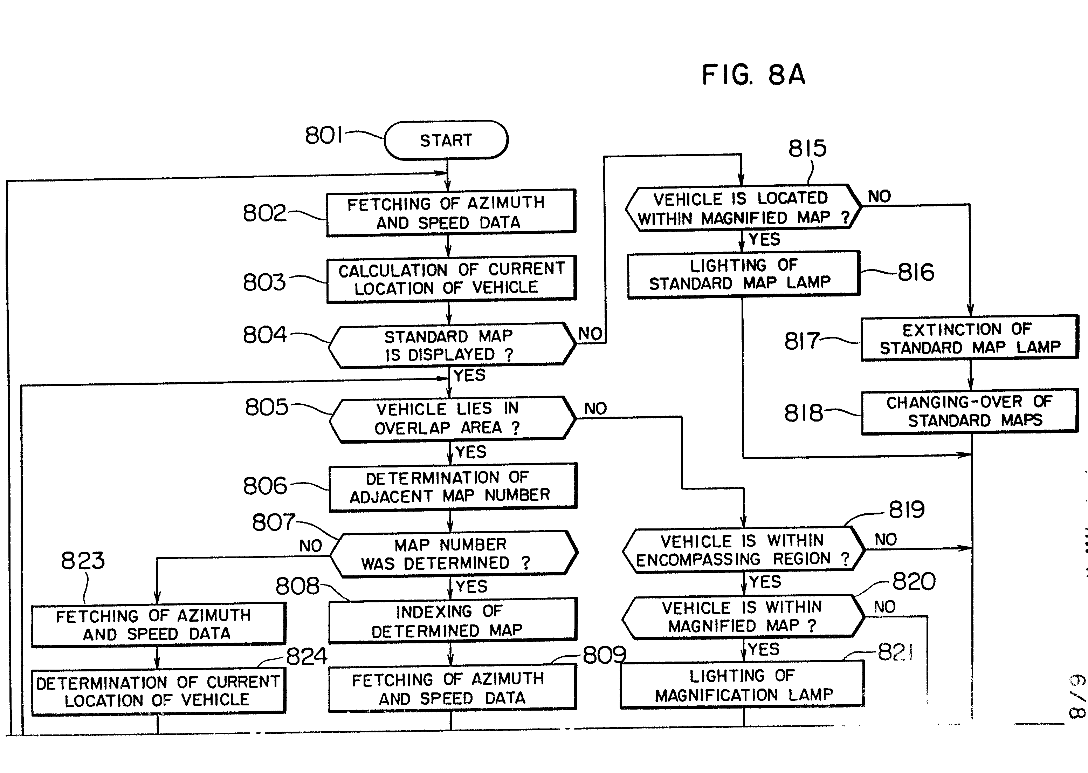

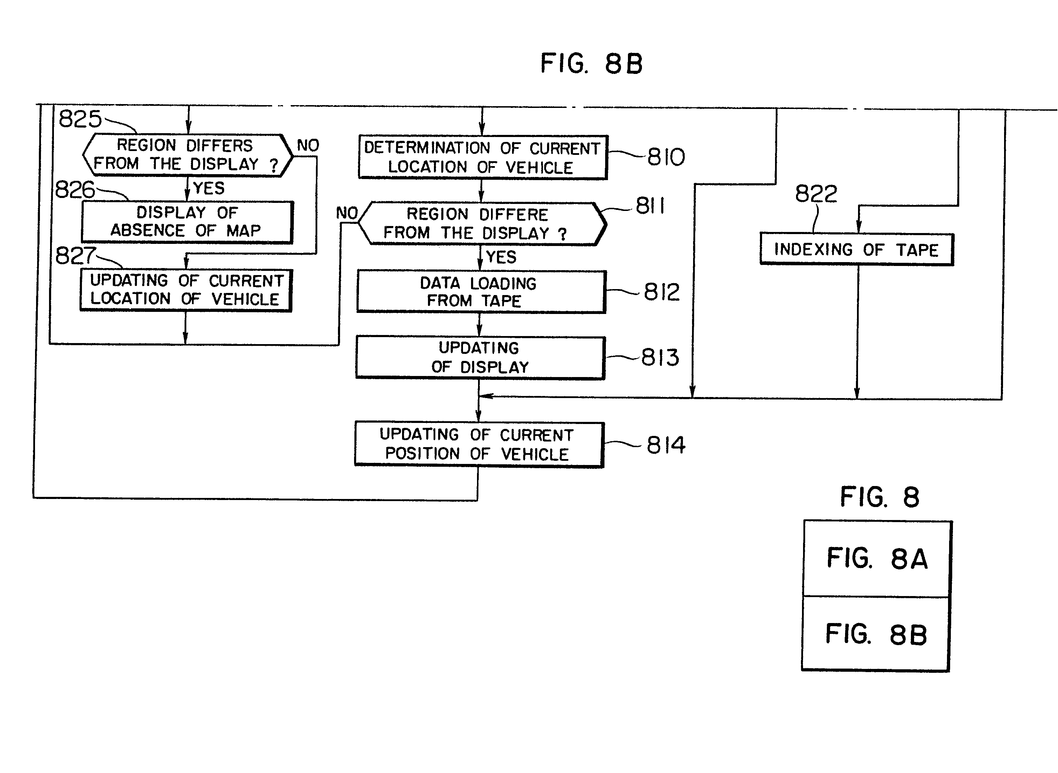

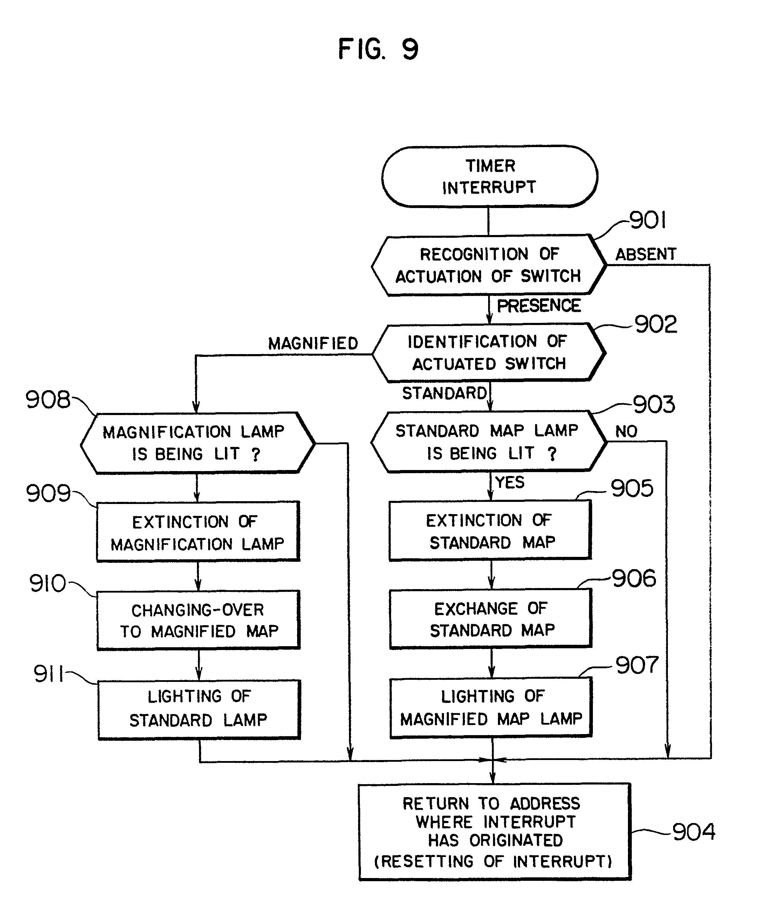

In the following, operation of the navigator system according to the illustrated embodiment of the invention will be described by referring to flow charg shown in Figs. 8 and 9.

After initialization of the navigator system has been completed by actuating the corresponding key switch shown in Fig. 2, a start step 810 is activated. At a step 802, data are fetched from the outputs of the azimuth or bearing sensor 101 and the vehicle speed sensor 102, which is followed by a step 803 where the position in the displayed map where the vehicle is currently located is arithmetically determined.

Next, at a step 804, it is checked whether the map being displayed on the CRT 116 is of the standard reduced scale or not. If the result is affirmative (YES), it is then checked whether the vehicle is located in an overlap area between the displayed map and adjacent one or whether the vehicle is located in an overlap area at one of the corners of the standard map being displayed for thereby determining the adjacent map to be displayed in consideration of the direction in which the vehicle trabels. In that case, the data contained in the section 704 is checked. The absence of the adjacent map as determined is indicated by the identification bit "0". Determination of the identification number of the adjacent map is made at a step 807. When the identification number of the adjacent map is determined, the corresponding road map data field of the cassette tape 105 is indexed.

Subsequently, the azimuth and speed data of the vehicle are fetched at a step 809, being followed by a step 810 where the updated current location of the vehicle is determined. At a step 812, it is checked whether the location of the vehicle is outside of the map being displayed. If so (YES), the adjacent road map data are read out from the cassette tape and loaded in the graphic memories 111, 112 and 113 under the control of the CMTC 106 at a step 812. At that time, the contents in the travel path memory 114 are altered in comformance with the new map. At a step 813, the map displayed on the CRT 116 is updated (i.e. exchanged with the new map). At the next step 814, the current location of the vehicle is loaded in the memory 114, whereupon the step 802 is regained.

When execution of the step 804 results in that the map being displayed is not of the standard reduced scale but a magnified map, it is then determined whether the updated location of the vehicle as determined at the step 803 lies inside of the map being displayed at a step 815. If so, the standard lamp 205 is lit at a step 816, and the displacement of the vehicle is added to the travel path. When it is decided at the step 815 that the vehicle is outside of the magnified map, the standard lamp 205 is turned off at a step 817. At the next step 818, the data of the preceding map of the standard reduced scale are again loaded in the graphic memories 111, 112 and 113, while the data of travel path placed in the memory 114 is replaced by the data for the standard map. At a step 814, the current location of the vehicle is updated, whereupon the step 802 is regained.

When it is decided at a step 805 that the vehicle is located outside of the overlap area between the standard map being displayed and an adjacent map, it is then checked whether the vehicle is located within the region covered by any one of the encompassing map 1 to i on the basis of the coordinate data 706 of the two corners of the encompassing map. If the vehicle is located in the magnified map, it is then checked at a step 802 whether the vehicle lies within the region covered by the magnified map. If so, the magnification lamp 204 is lit. On the contrary, when the vehicle is outside of the magnified map, the identification number of the magnified map is retrieved from the map number data attached to the encompassing map of the map data field, and the relevant road map data field of the cassette tape 102 is indexed. At the step 814, the current location of the vehicle is updated, whereupon the step 802 is regained.

Further, in case it is determined at the step 807 that the map of standard reduced scale is being displayed and the vehicle lies in the overlap area between the standard map and an adjacent map, while the identification number is not determined at the step 806, the azimuth and speed data are fetched at a step 823, being followed by a step 824 where the position of the vehicle is arithmetically determined. When it is found at a step 825 that the vehicle is outside of the map being displayed and running to the adjacent region for which the road map data are not available, dispaly of unavailability of the map is produced, whereupon the control procedure comes to an end. More specifically, the contents of the graphic memories 111, 112 and 113 for the road map data are inhibited from being displayed on the color CRT 116, while phrase data "NO MAP" stored in the ROM 108 is transferred to the memory 114 whose content is solely displayed on the CRT 116. When it is determined at the step 825 that the vehicle remains within the region being displayed, the current location of the vehicle is updated at a step 827 and the control procedure returns to the step 805.

Next, description will be made by referring to Fig. 9 which illustrates a flow of control procedure of pudating the display of the magnified map/standard map upon actuation of the magnification or standard push-button switches 204 or 205 shown in Fig. 2.

More specifically, when the control flow shown in Fig. 8 is performed, a timer interrupt is issued periodically at a predetermined time interval (e.g. 100 m sec.) so that the interrupt makes appearance in precedence to the start of the control procedure. In the interrupt routine, it is checked at a step 901 whether the magnification switch 204 or standard switch 205 shown in Fig. 2 is pushed down. When neigher switch key is actuated, the address or step (usually referred to us the return address) at which the interrupt has been issued in the course of execution of the control shown in Fig. 8 is regained.

When one of the switches 204 and 205 is actuated, it is determined at a step 902 whether the actuated one is the magnification switch 204 or the standard switch 205.

When the magnification switch is actuated, it is checked at a step 908 whether the lamp of the magnification switch 204 is lit or not. If not, actuation of the magnification switch 204 brings about no consequence. Accordingly, the return address is regained at a step 904, whereupon the interrupt is removed or reset. On the other hand, when the lamp of the switch 204 is lit, the lamp is put out at a step 909, being followed by a step 910 where the display is changed over to the magnified map. At the next step 911, the lamp of the standard switch 205 is lit, and the interrupt is removed at the step 904. The control procedure jumps to the return address.

When it is determined at the step 902 that the standard switch is actuated, it is checked at the step 903 whether the lamp of the standard switch 205 is lit or not. If not, the interrupt is removed and the return address is regained. If the lamp is lit, the lamp is put out at a step 905, being followed by a step 907 where the display is changed over to the standard map. After the lamp of the magnification switch 204 is lit at a step 907, the interrupt is cleared and jump is made to the return address.

The control operations described above is performed by the MPU 101, whereby the function to change over the display of standard map to that of the adjacent map or magnified map can be realized.

CLAIMS

1.      A navigator system for a motor vehicle, comprising a vehicle speed sensor (102) provided for determining a travel distance for which said vehicle has run on the basis of the speed of said vehicle; an azimuth sensor (101) for detecting the azimuth of the running direction of said vehicle; recording means (105) for storing road map digital information of a plurality of predetermined regions; control means (100) for selecting a map of the desired region from the map information stored in said recording means, determining the initial location of said vehicle in the selected map and updating the location of said vehicle in the displayed map on the basis of the data fetched from the outputs of said vehicle speed sensor and said azimuth sensor, as said vehicle runs; a manipulating switch panel (Fig.2) for commanding the selection of the map and determination of said initial location of said vehicle; and display means (115, 116) for displaying visually said selceted map, said intial location of said vehicle and the displacement of said vehicle by way of graphic memory means (111, 112, 113, 114);

wherein said recording means stores in addition to the data of standard maps at least the data which inidicate the presence or absence of adjacent map and the number of magnified maps together with data of said magnified map, said manipulating switch panel including switch means (202, 204) for selecting one of the standard map and the magnified map.

2.       A navigator system for a motor vehicle according to claim 1, wherein presence of a magnified map of a region for which the standard map is selected through actuation of said switch of said switch panel is displayed.

0169339

# FIG. 1

## FIG. 2

201 — NAVIGATOR

202 — SETTING          CANCEL — 203

204 — MAGNIFI-CATION   STANDARD — 205

206

208          △          207

◁    ▷

▽

209

## FIG. 3

302        301

TOWN A

HIGHWAY NO. 7

304          303

TOWN B

0169339

# FIG. 4

NAVIGATOR

SETTING   CANCEL

MAGNIFI-CATION   STAN-DARD

116

401

# FIG. 5

NAVIGATOR

SETTING   CANCEL

204   MAGNIFI-CATION   STAN-DARD

506   505

TOWN A

TOWN B

503

401

116

502

501

504

# FIG. 6

OVERLUP AREA BETWEEN M (m-1, n)
AND M (m+1, n)

| | | |
|---|---|---|
| 604<br><br>M (m+1, n-1) | 603<br><br>M (m+1, n) | 602<br><br>M(m+1, n+1) |
| 605<br><br>M(m, n-1) | 601<br><br>M(m, n) | 609<br><br>M (m, n+1) |
| 606<br><br>M(m-1, n-1) | 607<br><br>M(m-1, n) | 608<br><br>M(m-1, n-1) |

## FIG. 7

| MAP DATA M(m,n-1) | SEARCH DATA (m,n) | MAP DATA M(m,n) | SEARCH DATA M(m,n+1) |
|---|---|---|---|

701    702

| PRESENCE OR ABSENCE OF ADJACENT MAP | NUMBER OF ENCOMPASSING MAPS | COORDINATES OF TWO CORNERS OF ENCOMPASSING MAP 1 | MAP NO | ○○○○○ | COORDINATES OF TWO CORNERS OF ENCOMPASSING MAP ⊥ | MAP NO. | MAP DATA R.G.B M(m,n) |
|---|---|---|---|---|---|---|---|

705   706 707     703

| b7 b6 b5 b4 b3 b2 b1 b0 | 0 ~ 255 | X-COORDINATE OF LOWER LEFT CORNER | Y-COORDINATE OF LOWER LEFT CORNER | X-COORDINATE OF LOWER RIGHT CORNER | Y-COORDINATE OF LOWER LIGHT CORNER | m' | n' |
|---|---|---|---|---|---|---|---|
| ⌣ 1 BYTE ⌣ | ⌣ 1 ⌣ | ⌣ | 4 | | ⌣ | ⌣ 1 ⌣ | |

704    708 709 710    711 712

| PREAMBLE "0"x63 BIT | START "1" | LABEL A | DUMMY "1"x16 BIT | LABEL B | START "1" | PREAMBLE "0"x63 BIT |
|---|---|---|---|---|---|---|

# FIG. 8A

801 — START

802 — FETCHING OF AZIMUTH AND SPEED DATA

803 — CALCULATION OF CURRENT LOCATION OF VEHICLE

804 — STANDARD MAP IS DISPLAYED ? — NO / YES

805 — VEHICLE LIES IN OVERLAP AREA ? — NO / YES

806 — DETERMINATION OF ADJACENT MAP NUMBER

807 — MAP NUMBER WAS DETERMINED ? — NO / YES

823 — FETCHING OF AZIMUTH AND SPEED DATA

824 — DETERMINATION OF CURRENT LOCATION OF VEHICLE

808 — INDEXING OF DETERMINED MAP

809 — FETCHING OF AZIMUTH AND SPEED DATA

815 — VEHICLE IS LOCATED WITHIN MAGNIFIED MAP ? — NO / YES

816 — LIGHTING OF STANDARD MAP LAMP

817 — EXTINCTION OF STANDARD MAP LAMP

818 — CHANGING-OVER OF STANDARD MAPS

819 — VEHICLE IS WITHIN ENCOMPASSING REGION ? — NO / YES

820 — VEHICLE IS WITHIN MAGNIFIED MAP ? — NO / YES

821 — LIGHTING OF MAGNIFICATION LAMP

## FIG. 8B

825 — REGION DIFFERS FROM THE DISPLAY ? — NO

826 — DISPLAY OF ABSENCE OF MAP

827 — UPDATING OF CURRENT LOCATION OF VEHICLE

DETERMINATION OF CURRENT LOCATION OF VEHICLE — 810

811 — NO — REGION DIFFERE FROM THE DISPLAY ? — YES

DATA LOADING FROM TAPE — 812

UPDATING OF DISPLAY — 813

UPDATING OF CURRENT POSITION OF VEHICLE — 814

822 — INDEXING OF TAPE

## FIG. 8

| FIG. 8A |
|---------|
| FIG. 8B |

# FIG. 9

```
                    ┌─────────────┐
                    │    TIMER    │
                    │  INTERRUPT  │
                    └─────────────┘
                           │
                           ▼
            ┌──────────────────────────┐ ─901
            │     RECOGNITION OF       │  ABSENT
            │    ACTUATION OF SWITCH   │────────┐
            └──────────────────────────┘        │
                    │ PRESENCE                   │
                    ▼                            │
   MAGNIFIED ┌──────────────────────────┐ ─902  │
   ┌─────────│     IDENTIFICATION OF    │       │
   │         │      ACTUATED SWITCH     │       │
   │         └──────────────────────────┘       │
   │                 │ STANDARD                  │
   │                 ▼                           │
908─│         903─┌──────────────────────────┐  │
   ▼             │  STANDARD MAP LAMP       │ NO│
┌──────────────┐ │    IS BEING LIT ?        │───┤
│ MAGNIFICATION│ └──────────────────────────┘   │
│ LAMP         │        │ YES                    │
│ IS BEING LIT?│        ▼              905        │
└──────────────┘ ┌──────────────────┐           │
909─│           │   EXTINCTION OF   │           │
   ▼            │   STANDARD MAP    │           │
┌──────────────┐└──────────────────┘           │
│ EXTINCTION OF│        │                        │
│ MAGNIFICATION│        ▼              906        │
│ LAMP         │ ┌──────────────────┐           │
└──────────────┘ │   EXCHANGE OF    │           │
910─│           │   STANDARD MAP   │           │
   ▼            └──────────────────┘           │
┌──────────────┐        │                        │
│ CHANGING-OVER│        ▼              907        │
│ TO MAGNIFIED │ ┌──────────────────┐           │
│ MAP          │ │   LIGHTING OF    │           │
└──────────────┘ │ MAGNIFIED MAP LAMP│          │
911─│           └──────────────────┘           │
   ▼                                             │
┌──────────────┐                                 │
│ LIGHTING OF  │                                 │
│ STANDARD LAMP│                                 │
└──────────────┘                                 │
```

RECOGNITION OF ACTUATION OF SWITCH — 901 — ABSENT

IDENTIFICATION OF ACTUATED SWITCH — 902 — MAGNIFIED / STANDARD

MAGNIFICATION LAMP IS BEING LIT ? — 908

STANDARD MAP LAMP IS BEING LIT ? — 903 — NO / YES

EXTINCTION OF MAGNIFICATION LAMP — 909

EXTINCTION OF STANDARD MAP — 905

CHANGING-OVER TO MAGNIFIED MAP — 910

EXCHANGE OF STANDARD MAP — 906

LIGHTING OF STANDARD LAMP — 911

LIGHTING OF MAGNIFIED MAP LAMP — 907

RETURN TO ADDRESS WHERE INTERRUPT HAS ORIGINATED (RESETTING OF INTERRUPT) — 904

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | EP-A-0 066 877 (NIPPONDENSO)<br>* Abstract; figure 1; page 2, lines 1-12; page 4, lines 18-23; page 5, lines 4-10; page 20, lines 25-28 * | 1 | G 01 C 21/22<br>G 09 B 29/10 |
| | --- | | |
| A | GB-A-2 100 001 (NISSAN MOTOR CO. LTD.)<br>* Figures 6,7,8; page 5, lines 27-35 * | 1 | |
| | ----- | | |

|  |  |
|---|---|
| | **TECHNICAL FIELDS SEARCHED (Int Cl 4)** |
| | G 01 C<br>G 09 B |

The present search report has been drawn up for all claims

| Place of search | Date of completion of the search | Examiner |
|---|---|---|
| THE HAGUE | 25-09-1985 | HUNT J.H. |